# EUROPÄISCHE PATENTANMELDUNG

(11) **EP 4 803 109 A1**
(43) Veröffentlichungstag der Anmeldung: **09.09.2026**
(21) Anmeldenummer: 26162552.9
(22) Anmeldetag: 05.03.2026
(51) Int. Cl.: A61L 27/18, A61L 27/38, B33Y 80/00

(54) **SYNTHETISCHE BASALMEMBRAN UND VERFAHREN ZU IHRER HERSTELLUNG**

(30) Priorität: 07.03.2025 DE 102025108816
(71) Anmelder: Fraunhofer-Gesellschaft zur Förderung der angewandten Forschung e.V., 80686 München (DE)
(72) Erfinder: Weigel, Dr. Tobias, 97084 Würzburg (DE); Christ, Dr. Bastian, 97072 Würzburg (DE); Neser, Dr. Christina, 97980 Bad Mergentheim (DE); Wußmann, Dr. Maximiliane, 97078 Würzburg (DE); Appelt-Menzel, Dr. Antje, 97337 Dettelbach (DE)
(74) Vertreter: Gleiss Große Schrell und Partner mbB

(57) **Zusammenfassung**

Die vorliegende Erfindung betrifft Verfahren zur Herstellung einer künstlichen Basalmembran umfassend ein Fasergelege, künstliche Basalmembranen, umfassend ein Fasergelege, insbesondere hergestellt mittels des erfindungsgemäßen Verfahrens sowie in vitro-Testsysteme, Zellkultursysteme, Filtrationssysteme und Organsysteme, umfassend mindestens eine künstliche Basalmembran der vorliegenden Erfindung.

## Beschreibung

Die vorliegende Erfindung betrifft Verfahren zur Herstellung einer künstlichen Basalmembran umfassend ein Fasergelege, künstliche Basalmembranen, umfassend ein Fasergelege, insbesondere hergestellt mittels des erfindungsgemäßen Verfahrens sowie in vitro-Testsysteme, Zellkultursysteme, Filtrationssysteme und Organsysteme, umfassend mindestens eine künstliche Basalmembran der vorliegenden Erfindung.

Die Basalmembran ist ein zellfreies Gewebe mit einer Dicke von 1 bis 3 µm, welches aus fibrillären sowie retikulären Strukturproteinen aufgebaut ist. Die fibrillären Strukturen stellen die grundsätzliche Stabilität der Membran dar und sind hauptsächlich aus Kollagen Typ 3 aufgebaut. Zum stromalen Gewebe hin ist diese Struktur über weitere Kollagene wie Typ 7 mit dem angrenzenden Gewebe verankert. Auf der anderen Seite besteht die Basalmembran aus retikulären Strukturproteinen, hauptsächlich Kollagen Typ 4 und Laminine, welche ein dichtes Netzwerk bilden. Diese Struktur hat hauptsächlich die Funktion, spezifische Adhäsionssequenzen in hoher Dichte für Epithel- und Endothelzellen bereitzustellen. Generell ist diese Membran durchlässig für die meisten Moleküle und Makromoleküle und kann zudem von Immunzellen wie Makrophagen zur Abwehr von Pathogenen durchdrungen werden. Die Basalmembran stellt daher ein Grenzgewebe dar, welches zusammen mit der zellulären Komponente die Barriere in jedem Epithel und Endothel erzeugt.

In der Entwicklung von neuen pharmazeutischen Wirkstoffen ist die Aufnahmefähigkeit durch diese Barrieren von essentieller Bedeutung, da die biologische Verfügbarkeit im Körper entscheidend für die Wirksamkeit der immer komplexeren Wirkstoffe ist. Daher existiert eine stetig steigende Nachfrage an human physiologischen Gewebemodellen, welche die Barriere für Transportstudien nachbildet. Ebenso werden Barrieremodelle entwickelt, welche pathologische Eigenschaften, wie Infektionen, genetische Krankheiten oder Tumore abbilden und gleichermaßen in der Entwicklung von pharmazeutischen Wirkstoffen angewendet werden. Gleichzeitig geht es auch darum, Tierversuche zu reduzieren und potentere Alternativen anzubieten (in Anlehnung an das 3R-Prinzip). Das 3R-Prinzip steht für Reduce, Refine und Replace und somit für die Reduktion, das Verbessern und den Ersatz von Tierversuchen.

Für die Herstellung dieser Gewebe benötigen die Endothel- oder Epithelzellen die definierte dichte Struktur der Basalmembran sowie die Bereitstellung der Adhäsionssequenzen durch Kollagen Typ 4 und/oder der verschiedenen Laminine. Im Standard-Verfahren werden für diese Modelle "Tracked-Etched" Membranen als Zellkulturoberfläche verwendet, welche über ihre glatte Kunststoffoberfläche eine dichte Oberfläche bereitstellt und durch Poren mit definierten Größen im Bereich von wenigen 100 nm bis mehrere Mikrometer (je nach Applikation) die Durchlässigkeit von Kulturmedium und Wirkstoffen ermöglicht. Zur Bereitstellung der Zelladhäsionspunkte müssen diese Oberflächen aber zunächst mit Proteinen oder Peptiden/Peptidsequenzen aus der Basalmembran, insbesondere Kollagen Typ 4, Laminine, und Fibronektin, beschichtet werden. Daraus ergeben sich mehrere Nachteile, die primär das SubstratMaterial und dessen Proteinbeschichtung betreffen.

Das glatte Substratmaterial mit eingearbeiteten Poren hat den Nachteil, dass Moleküle nach Durchtritt durch die zelluläre Barriere nur eine geringe Anzahl an Penetrationen bzw. geringe Durchtrittsfläche zur Verfügung haben. Dadurch wird der Transport von Wirkstoffen durch das Barrierekonstrukt erheblich gebremst und gegebenenfalls in hohem Maße verhindert beziehungsweise verzögert und führt somit zu einer Unterschätzung der Bioverfügbarkeit des Wirkstoffes (Fehlerhafte Simulation der Aufnahme von Medikamenten). In manchen Fällen führt die hydrophobe Oberfläche sogar dazu, dass unspezifische Moleküladsorptionen am Membranmaterial Transportstudien unmöglich machen. In speziellen Anwendungen mit weiteren Zellen unterhalb des Barrieremodels reduzieren die geringe Anzahl und Größe an Poren ebenso zelluläre Interaktionen zwischen Barrierezellen und unterhalb angrenzender stromaler Zellen beziehungsweise Immunzellen.

Als Beschichtung dienen hierfür zum einen definierte Proteinlösungen aus Kollagen Typ 4 und/oder Lamininen und/oder Fibronektin, welche über unspezifische Adsorption die Materialoberfläche funktionalisieren. Nachteilig von definierten Proteinlösungen ist vor allem die aufwendige Herstellung und Aufreinigung und die damit verbundenen erheblichen Kosten. Zum anderen erfolgt die Funktionalisierung mit undefinierten Proteinlösungen: Diese Lösungen werden aus tierischen oder humanen Geweben hergestellt, welche einen hohen Anteil an Basalmembranproteinen aufweisen. Das am weitesten verbreitete Material ist Matrigel^{™}, welches in Tierversuchen aus künstlichen Tumoren isoliert wird. Dazu werden künstlich Tumore in Mäuse induziert und nach dessen Wachstum wieder entnommen, das Material verflüssigt, aufgereinigt und abschließend als Beschichtungslösung angeboten. Nachteilig ist dabei eine hohe Chargenvarianz, ebenso relativ hohen Kosten durch die aufwändige Herstellung, sowie jährliche Zahlen an verwendeten Tieren von weltweit etwa 300 000 Mäusen. Als Alternative erfolgen derzeit fortgeschrittene Entwicklungen, welche die Isolation der Basalmembranproteinen an Mäusen durch direkte humane Quellen ersetzen. Hierfür dient das Gewebe der Plazenta, welches bei der Geburt jedes Menschen anfällt und als Abfallprodukt für Aufbereitung und Isolation von EZM-Proteinen (extrazelluläre Matrix) zur Verfügung stehen würde. Nachteilig ist aber auch die Chargenvarianz durch genetische oder pathologische Unterschiede der jeweiligen menschlichen Proteine.

Ein weiterer Nachteil der Anwendung von Beschichtungslösungen für die Basalmembran ist, dass ausschließlich die qualitative und quantitative Bereitstellung der Adhäsionssequenzen erzielt werden kann. Das simple Prinzip der unspezifischen Adsorption der Proteine erzeugt damit eine zufällig generierte Bereitstellung der nötigen Peptidsequenzen, aber keine physiologische strukturelle Anordnung dieser Proteine zur strukturellen Nachahmung der Basalmembran.

Das der vorliegenden Erfindung zugrundeliegende technische Problem ist es, Verfahren zur Herstellung von Basalmembranen und damit hergestellte Basalmembranen bereitzustellen, welche die vorgenannten Nachteile bekannter Basalmembranen nicht aufweisen, insbesondere solche, welche es ermöglichen, funktional verbesserte in vitro-Testsysteme, Zellkultursysteme, Filtrationssysteme und Organsysteme bereitzustellen.

Dieses technische Problem wird gelöst durch die Bereitstellung der Lehren der unabhängigen und abhängigen Ansprüche sowie der dazugehörigen Beschreibung.

Dieses technische Problem wird insbesondere gelöst durch ein Verfahren zur Herstellung einer künstlichen Basalmembran umfassend ein Fasergelege, das in einer aus mindestens einem Basalmembranprotein aufgebauten Matrix eingebettet ist, umfassend die folgenden Verfahrensschritte:
a) Herstellen und Aufbringen eines Fasergeleges, umfassend Fasern mit einem Faserdurchmesser von 100 bis 2000 nm, mittels Elektrospinnen von einem Fasermaterial auf die Oberfläche eines in einem Elektrospinner angeordneten Trägerelements, wobei das Fasergelege eine Faserzahl von 1000 bis 30000 Fasern/mm² aufweist,
b) Aufbringen eines Stabilisierungsrahmens aus mindestens einem Polymer auf das auf dem Trägerelement angeordnete Fasergelege unter Bedingungen, die ein Assoziieren des Stabilisierungsrahmens mit dem Fasergelege erlauben,
c) Kultivieren des am Stabilisierungsrahmen assoziierten Fasergeleges mit in das Fasergelege eingebrachten Zellen mindestens eines Zelltyps, die zur Expression von mindestens einem Basalmembranprotein befähigt sind, unter Bedingungen, die die Expression des mindestens einen Basalmembranproteins erlauben und unter Erhalt einer aus dem mindestens einen Basalmembranprotein aufgebauten Matrix, in welcher das Fasergelege eingebettet ist und
d) Entfernen der Zellen des mindestens einen Zelltyps unter Erhalt der künstlichen Basalmembran.

Die vorliegende Erfindung sieht demgemäß vor, dass in einem ersten Verfahrensschritt a) mittels Elektronspinnen ein Fasergelege hergestellt wird, wobei dieses Fasergelege Fasern mit einem Faserdurchmesser von 100 bis 2000 nm aufweist. Verfahrensschritt a) wird dergestalt durchgeführt, dass das Elektrospinnen des Fasermaterials auf die Oberfläche eines in einem Elektrospinners angeordneten Trägerelementes erfolgt. Das Trägerelement ist ein auf einem Kollektor eines Elektrospinners abnehmbar angeordnetes, insbesondere flächiges, Element, das dem Auffangen und Trägern der gesponnenen Fasern dient. Die Oberfläche des Trägerelements ist eine definierte Oberfläche, das heißt eine Oberfläche festgelegter Größe und Geometrie. Verfahrensschritt a) wird ferner dergestalt durchgeführt, dass auf der definierten Oberfläche des Trägerelements ein Fasergelege mit einer Faserzahl (hier auch als Faserdichte bezeichnet) von 1000 bis 30000 Fasern/mm² hergestellt wird. Verfahrensschritt a) stellt also das Fasergelege her und bringt dieses gleichzeitig auf der Oberfläche eines Trägerelements auf. Die Erfindung sieht vor, einen Elektrospinnprozess mit einem definierten Material auf eine definierte Oberfläche so vorzusehen, dass eine genau definierte besonders geringe Faserdichte erreicht wird. In besonders vorteilhafter Weise wird so ein sehr dünnes Fasergelege mit geringer Faserdichte bereitgestellt, das im Rahmen der nachfolgenden Verfahrensschritte mit Basalmembranprotein-sezernierenden Zellen kontaktiert wird, die die Basalmembranproteine in und auf das Fasergelege abscheiden und eine das Fasergelege einbettende Matrix schaffen, die in ihrer Dicke in der Größenordnung natürlicher Basalmembranen liegt.

In einem Verfahrensschritt b) wird nach Aufbringen des Fasergeleges gemäß Verfahrensschritt a) ein Stabilisierungsrahmen auf dem auf dem Trägerelement befindlichen Fasergelege aufgebracht. Verfahrensschritt b) wird bevorzugt in oder mit einer Polymer-Aufbringvorrichtung durchgeführt, insbesondere in einer von dem Elektrospinner separaten Polymer-Aufbringvorrichtung. In einer Ausführungsform kann Verfahrensschritt b) mit einer Polymer-Aufbringvorrichtung auch in dem Elektrospinner durchgeführt werden. Der Stabilisierungsrahmen dient auch dazu, das erfindungsgemäße Fasergelege schadensfrei und formstabil von der Oberfläche des Trägerelements entnehmen zu können und so eine mechanische Stabilisierung, insbesondere bei der Entnahme, zu gewährleisten.

Das Aufbringen der Stabilisierungsrahmen auf das Fasergelege erfolgt unter Bedingungen, die ein Assoziieren des Stabilisierungsrahmens mit dem Fasergelege erlauben. Ein "Assoziieren" ist im Zusammenhang mit der vorliegenden Erfindung insbesondere eine, vorzugsweise reversible, Bindung des Stabilisierungsrahmens an das Fasergelege, die insbesondere durch Adhäsion erfolgt und stark genug ist, ein in einem Verfahrensschritt v) bevorzugt vorgesehenes Ablösen des an dem Stabilisierungsrahmen assoziierten Fasergeleges von der Oberfläche des Trägerelements zu ermöglichen. In einer Ausführungsform der Erfindung kann die Bindung des Stabilisierungsrahmens an das Fasergelege auch eine irreversible Bindung sein.

In einem anschließendem Verfahrensschritt c) wird das Fasergelege mit in das Fasergelege eingebrachten Zellen mindestens eines Zelltyps kultiviert, wobei dieser Zelltyp zur Expression von mindestens einem Basalmembranprotein, insbesondere zur Expression und Sezernierung mindestens eines Basalmembranproteins in die Zellumgebung, befähigt ist. Die Erfindung sieht in Verfahrensschritt c) also vor, Zellen des mindestens einen Zelltyps in und auf das Fasergelege einbeziehungsweise aufzubringen, also das mit dem Stabilisierungsrahmen assoziierte Fasergelege mit Zellen des mindestens einen Zelltyps in Kontakt zu bringen und die Zellen in dem Fasergelege zu kultivieren. In einer Ausführungsform kann auch das mit dem Trägerelement verbundene Konstrukt aus Fasergelege und Stabilisierungsrahmen mit Zellen des mindestens einen Zelltyps in Kontakt gebracht, die Zellen in dem Fasergelege kultiviert und den weiteren Verfahrensschritten unterzogen werden, gegebenenfalls unter dann erfolgender Ablösung des Trägerelements.

Verfahrensschritt c) wird unter Bedingungen durchgeführt, die die Expression dieses mindestens einen Basalmembranproteins in dem Zellkulturbehälter erlauben, insbesondere, die die Expression und Sezernierung des Basalmembranproteins aus der Zelle in seine Umgebung und damit in das Fasergelege erlauben, sodass in Verfahrensschritt c) ein Fasergelege erhalten wird, in dessen Faserzwischenräumen und auf dessen Faseroberflächen aus den Zellen sezernierte Basalmembranproteine ein- und aufgelagert sind. Diese Basalmembranproteine bilden so eine extrazelluläre Matrix mit in ihr eingebettetem Fasergelege aus.

Erfindungsgemäß wird es demgemäß also vorteilhafterweise ermöglicht, eine möglichst natürliche, physiologische Proteinzusammensetzung und -struktur bereitzustellen, die weitgehend der von natürlichen Basalmembranen entspricht. Erfindungsgemäß befindet sich diese in natürlicher Weise erzeugte Proteinkomponente der Basalmembran insbesondere in den Zwischenräumen und auf den Oberflächen des elektroversponnenen Materials, das heißt im und auf dem Fasergelege, und bildet so mit diesem Fasergelege eine integrale Hybridstruktur aus synthetisch hergestellten Fasern und diese umgebenden natürlich hergestellten Basalmembranproteinen. Die Zellen des mindestens einen Zelltyps werden daher besonders bevorzugt innerhalb der offenen und dünnen Fasergelege-Struktur, die gemäß der Verfahrensschritte a) und b) erhalten wurde, kultiviert und sezernieren die Basalmembranproteine daher auch innerhalb des Fasergeleges und nicht allein auf einer Oberfläche derselben. Erfindungsgemäß wird es dadurch möglich, eine Basalmembran zu erhalten, deren Proteinkomponenten nicht oberflächlich auf einem synthetischem Fasergelege aufliegen, sondern wobei das Fasergelege integraler Bestandteil der Basalmembranproteinstruktur ist und die Basalmembranproteine daher auch innerhalb des Fasergeleges vorliegen und so insgesamt nur eine geringe Dicke aufweist.

Die erfindungsgemäß bereitgestellten Basalmembranproteine können ebenso wie die in natürlichen Basalmembranen vorkommenden Proteine hinsichtlich ihrer Modifikationen wie Glykosylierungen genau den natürlicherweise vorkommenden Basalmembranproteinen entsprechen. Insbesondere werden erfindungsgemäß bevorzugt Zellen eingesetzt, welche retikuläre Basalmembranproteine wie Kollagen Typ 4, Laminine und Fibronektine produzieren. Die Kultivierung derartiger Zellen auf den Fasergelegen über eine Zeit, die ausreicht, die gewünschten Proteine zu exprimieren und sezernieren, erlaubt es innerhalb des elektroversponnenen, insbesondere dünnen, Fasergeleges, Proteine zu synthetisieren, die vollständig biologischer und somit natürlicher Herkunft sind und wobei diese ohne Verwendung von tierischen Materialien und Medien hergestellt werden können.

In einem anschließendem Verfahrensschritt d) werden die Zellen des mindestens einen Zelltyps entfernt, sodass die biologisch hergestellte Matrix und das in ihr eingebettete synthetisch hergestellte Fasergelege und damit die erfindungsgemäße Basalmembran zurückbleiben und erhalten werden.

Das erfindungsgemäße Verfahren stellt die erfindungsgemäße Basalmembran vorteilhafterweise unmittelbar unter Einsatz lebender Zellen zum Einbringen natürlicherweise hergestellter Basalmembranproteine in das, vorzugsweise sehr dünne, Fasergelege bereit, so dass die Basalmembran insbesondere aufgrund der in-vitro hergestellten Basalmembranproteine einer natürlicherweise vorkommenden Basalmembran und ihrer geringen Dicke sehr ähnlich ist.

Das erfindungsgemäße Verfahren erlaubt es vorteilhafterweise, eine besonders dünne Basalmembran herzustellen, die Menge an für die Herstellung eingesetzten künstlichen, also synthetischen Materials so gering wie möglich zu halten und durch die im Verfahren vorgesehene Expression der natürlichen Basalmembranproteine einen besonders hohen Anteil natürlichen Basalmembranproteins zu synthetischem Material zu erhalten.

Das in Verfahrensschritt a) eingesetzte Fasermaterial ist ein elektrospinnbares Material, insbesondere ein Polymer, insbesondere ein synthetisches oder an natürliches Polymer. Vorteilhafterweise und in bevorzugter Ausführungsform ist das Fasermaterial wasserunlöslich. In besonders bevorzugter Ausführungsform ist das Fasermaterial biokompatibel, das heißt insbesondere, dass es das Wachstum und die Funktionsfähigkeit von Zellen, insbesondere deren Fähigkeit, Proteine zu exprimieren und sie zu sezernieren, nicht beeinträchtigt.

In einer besonders bevorzugten Ausführungsform ist das Fasermaterial nicht biodegradierbar.

In einer besonders bevorzugten Ausführungsform ist das Fasermaterial biodegradierbar.

In einer besonders bevorzugten Ausführungsform ist vorgesehen, dass das in Verfahrensschritt a) eingesetzte Fasermaterial ausgewählt ist aus der Gruppe bestehend aus nicht degradierbaren Polymeren, insbesondere Polyether, Polyamide, Polyacrylnitril, Polystyrol, Polyethylenterephthalat, Polyoxazoline, chemisch funktionalisierte Polysaccharide und (Block-) Copolymere daraus, degradierbaren Polymeren, insbesondere Polyester, Poly-α-Hydroxysäuren, Polyglycolsäure, Polylactide, Polybutylsäuren, Polyurethane, Polypeptide und (Block-) Copolymere daraus, natürlichen Polymeren, insbesondere Polysacchariden, pflanzliche, tierische und menschliche Proteine, Lecithine, und anorganischen Materialien, insbesondere kondensiertes Tetraethylorthosilicat oder kondensierte Metallalkoholate, Silica-Gel, SiO₂, TiO₂, Bioglas und Kohlenstoff. In einer Ausführungsform können auch Kombinationen der genannten Substanzen versponnen werden.

In einer besonders bevorzugten Ausführungsform ist vorgesehen, dass das Fasermaterial Polyamid, Poly-α-Hydroxysäure oder Polyacrylnitril ist.

In einer besonders bevorzugten Ausführungsform können die Fasern einen Durchmesser von 200 bis 1500, insbesondere 300 bis 1000, insbesondere 300 bis 600 nm aufweisen.

Erfindungsgemäß bevorzugt sind die einzelnen Fasern des Fasergeleges untereinander frei beweglich und in besonders bevorzugter Ausführungsform nicht miteinander verbunden.

Das Elektroverspinnen des mindestens einen Fasermaterials in Verfahrensschritt a) kann bevorzugt mit einer Spannung von 6 kV bis 25 kV, insbesondere 8 bis 20 kV durchgeführt werden. Die relative Luftfeuchtigkeit (bei Raumtemperatur) beim Elektrospinnen kann bevorzugt 10 bis 90 %, insbesondere 20 bis 80 %, insbesondere 25 bis 50 % betragen, insbesondere so eingestellt werden.

Zusätzliche Polymere zum Elektroverspinnen des mindestens einen Fasermaterials in Verfahrensschritt a) können in bevorzugter Ausführungsform über separate Düsen oder Kanülen mit geeigneter Positionierung gleichzeitig und/oder abwechselnd elektroversponnen werden. Des Weiteren können zusätzliche Polymere direkt in der Lösung des mindestens einen Fasermaterials in Verfahrensschritt a) gelöst und als Polymermischung versponnen werden.

In einer besonders bevorzugten Ausführungsform der Erfindung ist vorgesehen, dass Verfahrensschritt a), das heißt das Elektrospinnen, über einen Zeitraum von 1 bis 30, vorzugsweise 2 bis 25, insbesondere 3 bis 20 min bezogen auf eine Faserquelle stattfindet.

In besonders bevorzugter Weise kann auch vorgesehen sein, die pro Fläche des Trägerelements aufgebrachte Materialmenge gezielt einzustellen, insbesondere durch Variation der Parameter Fläche, Spinnzeit, Förderrate und Konzentration des Polymers in der Spinnlösung.

In bevorzugter Ausführungsform beträgt die auf die Oberfläche des Trägerelements aufgebrachte Materialmenge der Fasern 10 bis 150 µg/cm², bevorzugt 18 bis 70 µg/cm² (jeweils bezogen auf Oberfläche des Trägerelements).

In einer bevorzugten Ausführungsform weist das Fasergelege Maschen mit einer durchschnittlichen Maschenfläche von maximal 400 µm², insbesondere maximal 200 µm², insbesondere maximal 150 µm² auf.

In weiterer bevorzugter Ausführungsform liegt die durchschnittliche Maschenfläche des Fasergeleges in einem Bereich von 10 bis 400 µm², bevorzugt 10 bis 200 µm², vorzugsweise 10 bis 150 µm², bevorzugt von 25 bis 120 µm².

In bevorzugter Ausführungsform beträgt die durchschnittliche Maschenfläche insbesondere 20 bis 200 µm², insbesondere 50 bis 200 µm², insbesondere 100 bis 200 µm², insbesondere 150 bis 200 µm², insbesondere 10 bis 150 µm², insbesondere 20 bis 150 µm², insbesondere 50 bis 150 µm², insbesondere 100 bis 150 µm², insbesondere 10 bis 100 µm², insbesondere 20 bis 100 µm², insbesondere 50 bis 100 µm², insbesondere 10 bis 50 µm², insbesondere 20 bis 50 µm².

In besonders bevorzugter Ausführungsform sieht die Erfindung vor, dass die Faserzahl pro Fläche von 1000 bis 30000 Fasern pro mm², insbesondere 1500 bis 25000 Fasern pro mm², insbesondere 2000 bis 20000 Fasern pro mm², beträgt.

In einer besonders bevorzugten Ausführungsform ist vorgesehen, dass das Fasergelege eine durchschnittliche Maschenfläche von 10 bis 200 µm² aufweist oder die auf die Oberfläche des Trägerelements aufgebrachte Materialmenge der Fasern 10 bis 150 µg/cm² Oberfläche des Trägerelementes beträgt, oder beides.

In einer besonders bevorzugten Ausführungsform ist vorgesehen, dass das Fasergelege 1 bis 20 µm, vorzugsweise 1,5 bis 10 µm, insbesondere 1 bis 5 µm, insbesondere 1 bis 3 µm, insbesondere 1,5 bis 3 µm, insbesondere bis 1 bis 2 µm oder 2 bis 3 µm dick ist. In einer besonders bevorzugten Ausführungsform ist vorgesehen, dass das Fasergelege 1 bis 3 µm dick ist.

In einer besonders bevorzugten Ausführungsform ist vorgesehen, dass das Fasergelege ohne Berücksichtigung optional vorhandener Stabilisierungsfasern 1 bis 20 µm, vorzugsweise 1,5 bis 10 µm, insbesondere 1 bis 5 µm, insbesondere 1 bis 3 µm, insbesondere 1,5 bis 3 µm, insbesondere bis 1 bis 2 µm oder 2 bis 3 µm dick ist. In einer besonders bevorzugten Ausführungsform ist vorgesehen, dass das Fasergelege ohne Berücksichtigung optional vorhandener Stabilisierungsfasern 1 bis 3 µm dick ist.

In einer besonders bevorzugten Ausführungsform ist vorgesehen, dass das Fasergelege unter Berücksichtigung optional vorhandener Stabilisierungsfasern 1 bis 20 µm, vorzugsweise 1,5 bis 10 µm, insbesondere 1 bis 5 µm, insbesondere 1 bis 3 µm, insbesondere 1,5 bis 3 µm, insbesondere bis 1 bis 2 µm oder 2 bis 3 µm dick ist. In einer besonders bevorzugten Ausführungsform ist vorgesehen, dass das Fasergelege unter Berücksichtigung optional vorhandener Stabilisierungsfasern 1 bis 3 µm dick ist.

In einer besonders bevorzugten Ausführungsform ist das Trägerelement eine flächige Metallstruktur, insbesondere Metallfolie, insbesondere eine Aluminiumfolie.

In besonders bevorzugter Ausführungsform kann vorgesehen sein, dass die Oberfläche des zu bespinnenden Trägerelements mit einem Modifikationsmaterial, insbesondere einem wasserlöslichen Material beschichtet ist. Dies kann vorteilhafterweise den Effekt haben, dass so eine elektrostatische Anheftung der elektroversponnenen Fasern an das Trägerelement verhindert oder reduziert wird.

In einer besonders bevorzugten Ausführungsform ist vorgesehen, dass die Oberfläche des Trägerelementes vor Verfahrensschritt a) in einem Verfahrensschritt x) mit einem wasserlöslichen Modifikationsmaterial beschichtet wird.

In einer besonders bevorzugten Ausführungsform kann das wasserlösliche Modifikationsmaterial ein wasserlösliches Polymer, ein wasserlösliches Saccharid oder ein wasserlösliches anorganisches Material sein.

In besonders bevorzugter Ausführungsform kann das wasserlösliche Modifikationsmaterial eine Beschichtung, ein Film oder eine partikuläre Beschichtung sein. Insbesondere kann das wasserlösliche Modifikationsmaterial ein wasserlösliches Polymer, insbesondere Polyvinylalkohol, Polyethylenglykol, Polyvinylpyrrolidon, Polyacrylamid, Polyacrylsäure oder eine Mischung derselben sein. In besonders bevorzugter Ausführungsform kann das wasserlösliche Modifikationsmaterial auch ein wasserlösliches Saccharid, zum Beispiel Oligosaccharid, sein. In weiterer bevorzugter Ausführungsform kann das wasserlösliche Modifikationsmaterial auch ein anorganisches Material, zum Beispiel ein wasserlösliches Salz, insbesondere Natriumchlorid oder andere Natrium-, Kalium- und Nitratsalze sein.

In einer besonders bevorzugten Ausführungsform ist vorgesehen, dass im Anschluss an Verfahrensschritt a) das das Fasergelege aufweisende Trägerelement in einem Verfahrensschritt z) aus dem Elektrospinner entnommen und in eine Vorrichtung zum Durchführen von Verfahrensschritt b) überführt wird.

In einer besonders bevorzugten Ausführungsform wird in Verfahrensschritt b) der Stabilisierungsrahmen als Zellkulturbehälter ausgebildet. In bevorzugter Ausführungsform wird in Verfahrensschritt b) also ein Stabilisierungsrahmen aufgebracht, der dazu ausgebildet, eingerichtet und geeignet ist, eine Kultivierung von Zellen gemeinsam mit dem Fasergelege zu erlauben. In dieser Ausführungsform dient der Stabilisierungsrahmen im nachfolgenden Verfahrensschritt c) dem Kultivieren der Zellen des mindestens einen Zelltyps.

In einer besonders bevorzugten Ausführungsform wird in Verfahrensschritt b) der Stabilisierungsrahmen als Zellkulturbehälter ausgebildet und das in Verfahrensschritt c) vorgesehene Kultivieren der Zellen in dem als Zellkulturbehälter ausgebildeten Stabilisierungsrahmen durchgeführt.

Bevorzugt ist die Vorrichtung zum Durchführen von Verfahrensschritt b) eine Polymer-Aufbringvorrichtung, insbesondere ein Drucker oder Sprüher.

Der Stabilisierungsrahmen ist bevorzugt aus einem oder mehreren Polymeren aufgebaut. In einer besonders bevorzugten Ausführungsform ist das Polymer ein biokompatibles Polymer, insbesondere ein Thermoplast, insbesondere Polycaprolacton (PCL), Polyetheretherketon (PEEK), Polylactid (PLA), Polyetherimide (PEI), PE (Polyethylen), PP (Polypropylen), PC (Polycarbonat), PS (Polystyrol), PET (Polyethylenterephthalat), PA (Polyamid), POM (Polyoxymethylen), PGA (Polyglykolsäure), PHB (Polyhydroxybuttersäure) oder Poly(methylmethacrylat) (PMMA) oder eine Kombination davon.

In einer besonders bevorzugten Ausführungsform ist vorgesehen, dass der Stabilisierungsrahmen in Verfahrensschritt b) mittels eines Druckverfahrens auf das Fasergelege aufgebracht wird, insbesondere auf dessen Oberseite, also die von der Oberfläche des Trägerelements abgewandte Seite.

In einer besonders bevorzugten Ausführungsform kann das Druckverfahren unter Einsatz eines 3D-Druckers erfolgen.

In einer besonders bevorzugten Ausführungsform kann das Material des Stabilisierungsrahmens mittels eines Fused-Deposition-Modellings (FDM) auf das Fasergelege aufgebracht werden, bevorzugt unter Anwendung von Thermoplasten, insbesondere biokompatiblen Materialien, für den dreidimensionalen Druck.

Erfindungsgemäß ist es bevorzugt, dass der in Verfahrensschritt b) aufgebrachte Stabilisierungsrahmen individuell auf die Größe und Form der für die hergestellte künstliche Basalmembran vorgesehenen Anwendung angepasst ist. In bevorzugter Ausführung kann der Stabilisierungsrahmen die Form und Größe eines Transwell-Systems, eines Bioreaktors oder eines Organ/Human-on-a-chip-Systems aufweisen. Der Stabilisierungsrahmen kann in bevorzugter Ausführungsform ringförmig, also zum Beispiel ein Stabilisierungsring, oder eckig, insbesondere rechteckig, sein.

In besonders bevorzugter Ausführungsform weist der Stabilisierungsrahmen eine Breite von 100 bis 1000 µm, vorzugsweise 200 bis 800 µm, und eine Dicke von 50 bis 600 µm, insbesondere 100 bis 500 µm auf.

In einer besonders bevorzugten Ausführungsform wird zeitlich nach Verfahrensschritt b) in einem Verfahrensschritt v) das mit dem Stabilisierungsrahmen assoziierte Fasergelege von dem Trägerelement abgelöst, insbesondere vor Durchführung von Verfahrensschritt c), nach Durchführung von Verfahrensschritt c), vor Durchführung von Verfahrensschritt d) oder nach Durchführung von Verfahrensschritt d).

Das in Verfahrensschritt v) bevorzugt vorgesehene Ablösen des Stabilisierungsrahmens mit dem anheftenden Fasergelege von dem Trägerelement kann insbesondere durch Inkubation in wässriger Lösung, insbesondere Wasser, zum Beispiel für 0,5 bis 5 Minuten, insbesondere 0,5 bis 1 min, erfolgen.

In bevorzugter Ausführungsform ist in einem Verfahrensschritt v) vorgesehen, dass vor dem Kultivieren des mit dem Stabilisierungsrahmen assoziierten Fasergeleges in Verfahrensschritt c) zunächst ein Ablösen des mit dem Stabilisierungsrahmen assoziierten Fasergeleges von der Oberfläche des Trägerelements erfolgt.

In einer besonders bevorzugten weiteren Ausführungsform erfolgt in einem an Verfahrensschritt b) anschließenden Verfahrensschritt, hier auch als Verfahrensschritt w) bezeichnet, das Überführen des mit dem Stabilisierungsrahmen assoziierten Fasergeleges in einen Zellkulturbehälter. Je nachdem in welcher Vorrichtung das Aufbringen des Stabilisierungsrahmens stattgefunden hat, erfolgt das Überführen entweder aus dem Elektrospinner oder aus der Polymer-Aufbringvorrichtung.

In bevorzugter Ausführungsform ist in einem Verfahrensschritt v) vorgesehen, dass vor dem in Verfahrensschritt w) vorgesehenen Überführen des mit dem Stabilisierungsrahmen assoziierten Fasergeleges in den Zellkulturbehälter zunächst ein Ablösen des mit dem Stabilisierungsrahmen assoziierten Fasergeleges von der Oberfläche des Trägerelements erfolgt, so dass lediglich das am Stabilisierungsrahmen fixierte Fasergelege überführt wird, ohne das Trägerelement.

Bevorzugt im Anschluss an das in Verfahrensschritt v) vorgesehene Ablösen des mit dem Stabilisierungsrahmen verbundenen Fasergeleges von dem Trägerelement wird dieses Konstrukt in einem Verfahrensschritt w) in einen Zellkulturbehälter überführt und in dem anschließenden Verfahrensschritt c) in dem Zellkulturbehälter mit in das Fasergelege eingebrachten Zellen mindestens eines Zelltyps kultiviert, wobei dieser Zelltyp zur Expression von mindestens einem Basalmembranprotein, insbesondere zur Expression und Sezernierung mindestens eines Basalmembranproteins in die Zellumgebung, befähigt ist. In einer Ausführungsform kann auch das mit dem Trägerelement verbundene Konstrukt aus Fasergelege und Stabilisierungsrahmen in den Zellkulturbehälter überführt werden und den weiteren Verfahrensschritten unterzogen werden, gegebenenfalls unter dann erfolgender Ablösung des Trägerelements.

In einer besonders bevorzugten Ausführungsform wird also im Anschluss an Verfahrensschritt b) das mit dem Stabilisierungsrahmen assoziierte Fasergelege in einem Verfahrensschritt w) in einen separaten Zellkulturbehälter überführt und in dem separaten Zellkulturbehälter Verfahrensschritt c) durchgeführt.

In einer weiteren bevorzugten Ausführungsform kann vorgesehen sein, das Fasergelege auf einer seiner Seiten, insbesondere Ober- oder Unterseite, zu stabilisieren.

In einer besonders bevorzugten Ausführungsform ist vorgesehen, dass vor Verfahrensschritt c) in einem Verfahrensschritt y) Stabilisierungsfasern mit einem Faserdurchmesser von 1 bis 500 µm aus einem Stabilisierungsmaterial auf mindestens eine Seite, insbesondere die Oberseite oder Unterseite oder beide Seiten des Fasergeleges aufgebracht werden.

In einer weiteren bevorzugten Ausführungsform kann vorgesehen sein, das Fasergelege auf seiner Unterseite durch Aufbringen von Stabilisierungsfasern zu stabilisieren, das heißt auf der Seite, auf der nicht der Stabilisierungsrahmen aufliegt.

In einer weiteren bevorzugten Ausführungsform kann vorgesehen sein, das Fasergelege auf seiner Oberseite durch Aufbringen von Stabilisierungsfasern zu stabilisieren, das heißt auf der Seite, auf der der Stabilisierungsrahmen aufliegt.

In besonders bevorzugter Ausführungsform kann der Faserdurchmesser der Stabilisierungsfasern in einem Bereich von 1 bis 500 µm, insbesondere 5 bis 200 µm, insbesondere 5 bis 20 µm liegen.

In besonders bevorzugter Ausführungsform können die Stabilisierungsfasern aus Polyamid, Polycaprolacton oder Polyester bestehen oder dieses umfassen.

In besonders bevorzugter Ausführungsform können kommerziell erhältliche Netze, insbesondere aus Polyamid oder Polyester, aufgebracht werden.

In besonders bevorzugter Ausführungsform können die Stabilisierungsfasern durch direktes Aufbringen der Fasern mittels Mikrofaserspinnverfahren, zum Beispiel Melt-Electro-Writing, Druckspinnen, Melt-Blow-Spinnen oder Faserziehen aufgebracht werden.

In einer besonders bevorzugten Ausführungsform ist vorgesehen, dass die Stabilisierungsfasern in einem Verfahrensschritt y1) im Anschluss an Verfahrensschritt a) und vor Verfahrensschritt b) auf die Oberseite des auf dem Trägerelements befindlichen Fasergeleges aufgebracht werden.

In einer besonders bevorzugten Ausführungsform ist vorgesehen, dass die Stabilisierungsfasern in einem Verfahrensschritt y2) im Anschluss an das in Verfahrensschritt v) durchgeführte Ablösen des am Stabilisierungsrahmen assoziierten Fasergeleges von dem Trägerelement und vor dem in Verfahrensschritt w) bevorzugt vorgesehenen Überführen in einen Zellkulturbehälter auf die Unterseite des Fasergeleges aufgebracht werden.

In besonders bevorzugter Ausführungsform sind die in Verfahrensschritt c) kultivierten Zellen mindestens eines Zelltyps Zellen, die in der Lage sind, Proteine der Basalmembran, insbesondere Strukturproteine, insbesondere Proteoglykane, insbesondere Kollagen Typ 4, Laminine, Fibronektin, oder eine Kombination davon zu exprimieren und sezernieren.

In besonders bevorzugter Ausführungsform sind die Zellen mindestens eines Zelltyps Zellen, die in der Lage sind, fibrilläre Strukturproteine wie Kollagen Typ 1, 3 oder Typ 7 zu exprimieren und sezernieren.

In besonders bevorzugter Ausführungsform können die fibrilläre Kollagene bereitstellenden humanen Zelllinien MCF-8, HeLa, HUVEC, HEK203, L929, A549, Caco-2 oder MDCK sein.

In einer besonders bevorzugten Ausführungsform können die fibrilläre Kollagene bereitstellenden Zellen humane, stromale Zelllinien sein, insbesondere HFF-1, MRC-5, HS-27A, HS-5 oder NGH-3T3.

In einer besonders bevorzugten Ausführungsform können die fibrilläre Kollagene bereitstellenden Zellen induzierte pluripotente Stammzellen (iPS), insbesondere IMR90-4, UKBi005 oder UKKi011 sein.

Darüber hinaus können primäre humane Zelllinien eingesetzt werden, welche in der Lage sind, Col3 (Typ 3 Kollagen) zu exprimieren und sezernieren. Darüber hinaus können zu diesem Zweck primäre humane, stromale Zelllinien, iPS-abgeleitete Stromalzellen und tierische Stromalzellen, insbesondere auch Zelllinien eingesetzt werden.

In besonders bevorzugter Ausführungsform sind die Zellen mindestens eines Zelltyps Zellen, die in der Lage sind, retikuläre Strukturproteine wie Kollagen Typ 4, Fibronektine oder Laminine zu exprimieren und sezernieren.

In einer besonders bevorzugten Ausführungsform können endotheliale Zellen, epitheliale Zellen oder Tumorzellen verwendet werden.

In einer besonders bevorzugten Ausführungsform können die Zellen Zellen aus humanen Zelllinien sein, primäre humane Zellen, iPS-abgeleitete Zellen oder tierische Zellen.

In einer besonders bevorzugten Ausführungsform können die humanen Zelllinien Zelllinien aus Gigablastomen oder Zelllinien aus anderen Karzinomen, insbesondere aus Brust-, Lungen-, Prostata-, Magenkrebs oder invasiven oder metastasierenden Melanomen sein. In besonders bevorzugter Ausführungsform kann die humane Zelllinie aus dem Epithel sein. In besonders bevorzugter Ausführungsform kann die Zelllinie aus dem Endothel sein.

In besonders bevorzugter Ausführungsform können die primären Humanzellen Tumorzellen, Epithel- oder Endothelzellen sein. In einer besonders bevorzugten Ausführungsform können die iPS-abgeleiteten Zellen Tumor-, Epithel- und/oder Endothelzellen sein. In besonders bevorzugter Ausführungsform können die tierischen Zellen auch tierische Zelllinien sein, insbesondere Tumorzellen, Epithel- und Endothelzellen.

In einer besonders bevorzugten Ausführungsform ist vorgesehen, dass der Zelltyp ausgewählt ist aus der Gruppe bestehend aus humanen Zelllinien, tierischen Zelllinien, primären humanen Zellen, primären tierischen Zellen, induzierten pluripotenten Zelllinien (IPS), IPS-abgeleiteten Zellen und stromalen Zellen.

In einer besonders bevorzugten Ausführungsform erfolgt die Kultivierung für einen Zeitraum von 5 bis 30, insbesondere 7 bis 21 Tagen.

In einer besonders bevorzugten Ausführungsform ist vorgesehen, dass Verfahrensschritt c) mindestens zweimal sequentiell mit Zellen unterschiedlicher Zelltypen durchgeführt wird.

In besonders bevorzugter Ausführungsform wird ein Verfahren bereitgestellt, in dem eine zweischichtige Basalmembran hergestellt wird. In besonders bevorzugter Ausführungsform kann vorgesehen sein, in einem Verfahrensschritt c) in einer ersten Phase Zellen mindestens eines ersten Zelltyps und anschließend in einer zweiten Phase Zellen mindestens eines zweiten Zelltyps zu kultivieren, sodass in den zwei unterschiedlichen Phasen unterschiedliche Basalmembranproeine in dem Fasergelege abgeschieden werden.

In einer besonders bevorzugten Ausführungsform kann in einer ersten Phase des Verfahrensschritts c) vorgesehen sein, Zellen eines ersten Zelltyps zu kultivieren, die eine Bereitstellung von fibrillären Proteinen, insbesondere Kollagen Typ 3 oder Kollagen Typ 1, ermöglichen und in einer zweiten Phase Zellen eines zweiten Zelltyps zu kultivieren, die eine Bereitstellung von retikulären Proteinen ermöglichen, insbesondere Kollagen Typ 4 oder Laminine. Aufgrund der zeitlichen Abfolge erfolgt so in der zweiten Phase eine Bildung einer retikulären Proteinschicht oberhalb der während der ersten Phase eingebrachten fibrillären Proteine.

In einer besonders bevorzugten Ausführungsform kann das in Verfahrensschritt d) vorgesehene Entfernen der Zellen des mindestens einen Zelltyps unter Erhalt der künstlichen Basalmembran durch Inkubation in Dezellularisierungs-Lösungen erfolgen, insbesondere in Lösungen mit den Agentien Natriumdodecylsulfat, Natriumdesoxycholat, Triton X-100, Tri-n-Butylphosphat (TnBP), CHAPS (3-[(3-cholamidopropyl) dimethylammonio]-1-propansulfonat) oder hypertonischen oder hypotonischen Lösungen,.

Vorzugsweise werden Dezellularisierungs-Lösungen eingesetzt, wobei die dezellularisierenden Agentien in einer Konzentration von 0,1 bis 3 Vol.-% vorliegen.

In einer besonders bevorzugten Ausführungsform beträgt die Inkubationsdauer für die Dezellularisierung von 0,5 bis 5 Stunden (h).

In einer besonders bevorzugten Ausführungsform kann vorgesehen sein, die nach der Dezellularisierung möglicherweise in dem erfindungsgemäß hergestellten Produkt vorhandenen Zellfragmente durch ein bis mehrfaches Spülen, insbesondere mit physiologischer Salzlösung auszuwaschen und gegebenenfalls verbleibende DNA durch insbesondere 0,5- bis zweistündige, insbesondere einstündige, Inkubation in DNase-Lösung abzubauen.

In einer Ausführungsform der Erfindung kann der Stabilisierungsrahmen im Anschluss an Verfahrensschritt c), insbesondere im Anschluss an Verfahrensschritt d), entfernt werden.

In einer besonders bevorzugten Ausführungsform ist vorgesehen, dass die künstliche Basalmembran 1 bis 20 µm, vorzugsweise 1,5 bis 10 µm, insbesondere 1 bis 5 µm, insbesondere 1 bis 3 µm, insbesondere 1,5 bis 3 µm, insbesondere 1 bis 2 µm oder 2 bis 3 µm dick ist.

In einer besonders bevorzugten Ausführungsform ist vorgesehen, dass die künstliche Basalmembran 1 bis 3 µm dick ist.

In einer besonders bevorzugten Ausführungsform ist vorgesehen, dass die künstliche Basalmembran ohne Berücksichtigung optional vorhandener Stabilisierungsfasern 1 bis 20 µm, vorzugsweise 1,5 bis 10 µm, insbesondere 1 bis 5 µm, insbesondere 1 bis 3 µm, insbesondere 1,5 bis 3 µm, insbesondere 1 bis 2 µm oder 2 bis 3 µm dick ist.

In einer besonders bevorzugten Ausführungsform ist vorgesehen, dass die künstliche Basalmembran ohne Berücksichtigung optional vorhandener Stabilisierungsfasern 1 bis 3 µm dick ist.

In einer besonders bevorzugten Ausführungsform ist vorgesehen, dass die künstliche Basalmembran unter Berücksichtigung optional vorhandener Stabilisierungsfasern 1 bis 20 µm, vorzugsweise 1,5 bis 10 µm, insbesondere 1 bis 5 µm, insbesondere 1 bis 3 µm, insbesondere 1,5 bis 3 µm, insbesondere 1 bis 2 µm oder 2 bis 3 µm dick ist.

In einer besonders bevorzugten Ausführungsform ist vorgesehen, dass die künstliche Basalmembran unter Berücksichtigung optional vorhandener Stabilisierungsfasern 1 bis 3 µm dick ist.

Die vorliegende Erfindung stellt auch eine künstliche Basalmembran bereit, umfassend ein Fasergelege aus elektroversponnenen Fasern mit einem Faserdurchmesser von 100 bis 2000 nm, wobei das Fasergelege in einer von Zellen mindestens eines Zelltyps, die zur Expression von mindestens einem Basalmembranprotein befähigt sind, erzeugten Matrix eingebettet ist, insbesondere hergestellt mittels eines erfindungsgemäßen Verfahrens.

Die vorliegende Erfindung stellt auch ein in vitro-Testsystem, Zellkultursystem, künstliches Gewebe, Implantat, Insert-System, Filtrationssystem und Human-on-a-chip-/Organsystem bereit, umfassend mindestens eine erfindungsgemäße Basalmembran.

Die vorliegende Erfindung stellt insbesondere ein in vitro-Testsystem, insbesondere Barrieremodell, bereit, umfassend mindestens eine erfindungsgemäße Basalmembran.

Die erfindungsgemäße Basalmembran kann zell-frei oder zell-haltig verwendet werden.

Erfindungsgemäß bevorzugt kann die Basalmembran in zell-freier oder zell-haltiger Ausführung als Insertsystem oder als Organ-/ beziehungsweise Human-on-a-chip-System angewendet werden, insbesondere in Anwendung im Bereich der beziehungsweise des:
Blut-Hirn-Schranke/Neurovaskuläre Einheit, Darm/Gastrointestinaltrakts, Endothel, Lunge, Atemwege, Haut, Mundschleimhaut, Cornea, Retinal-pigmentiertes Epithel, Pathologische Varianten der genannten Modelle, Leber, Niere, alle weiteren inneren Organe.

Die erfindungsgemäße Basalmembran kann in vitro oder in vivo, zum Beispiel als zell-freies oder zell-haltiges, Implantat verwendet werden.

Die erfindungsgemäße Basalmembran kann auch als Filtrationssystem, insbesondere Filter oder Filtermaterial, insbesondere für Sterilfiltrationen eingesetzt werden.

Die erfindungsgemäße Basalmembran kann auch für die Stammzell-Kultur oder die Kultur und Anwendung von Spheroiden, Organoiden und Assembloiden verwendet werden.

In einer besonders bevorzugten Ausführungsform umfasst die künstliche Basalmembran in oder auf die künstliche Basalmembran aufgebrachte Zellen mindestens eines Ziel-Zelltyps.

Zellen eines Ziel-Zelltyps sind Zellen, die auf oder in eine erfindungsgemäße Basalmembran eingebracht und kultiviert werden, um so eine erwünschte Zell-basierte Anwendbarkeit unter Einsatz von kultivierten Zellen, zum Beispiel als Testsystem, insbesondere in vitro-Testsystem, insbesondere Barrieremodell, oder als künstliches Gewebe- oder Organsystem bereitzustellen.

Zellen mindestens eines Ziel-Zelltyps können in bevorzugter Ausführungsform Dünndarmzellen, Epithelzellen, Endothelzellen oder Tumorzellen sein.

In einer bevorzugten Ausführungsform der Erfindung ist der mindestens eine Ziel-Zelltyp ausgewählt aus der Gruppe bestehend aus Fibroblasten, insbesondere cancer associated fibroblasts, Keratinozyten, mesenchymale Stammzellen, iPS-Zellen (induziert pluripotente Stammzellen), Tumorzellen, Endothelzellen und Kombinationen davon.

Die Erfindung betrifft auch die Verwendung einer erfindungsgemäßen Basalmembran zur Kultivierung oder Differenzierung von Zellen.

Die Erfindung betrifft auch ein künstliches Gewebe umfassend mindestens eine erfindungsgemäße Basalmembran und mindestens eine Zelle mindestens eines Ziel-Zelltyps.

In einer bevorzugten Ausführungsform weist das künstliche Gewebe mehrere, insbesondere mindestens zwei, insbesondere mindestens drei, erfindungsgemäße Basalmembranen auf.

Die Erfindung betrifft auch ein künstliches Gewebe, umfassend eine erfindungsgemäße Basalmembran und Zellen mindestens eines Ziel-Zelltyps, wobei das künstliche Gewebe 1 bis 50 Gew.-% Basalmembran und 50 bis 99 Gew.-% Zellen, insbesondere 80 bis 99 Gew.-% Zellen umfasst (jeweils bezogen auf das Gesamtgewicht des künstlichen Gewebes).

In bevorzugter Ausführungsform ist das künstliche Gewebe ein Dünndarm-Modell.

In bevorzugter Ausführungsform ist das künstliche Gewebe ein Modell einer Blut-Hirn-Schranke/Neurovaskulären Einheit.

In bevorzugter Ausführungsform ist das künstliche Gewebe ein Hautmodell, insbesondere umfassend eine Dermis, umfassend mindestens eine erfindungsgemäße Basalmembran. In bevorzugter Ausführungsform ist das künstliche Gewebe ein Hautmodell, bevorzugt umfassend eine Subcutis umfassend mindestens eine erfindungsgemäße Basalmembran.

In bevorzugter Ausführungsform ist das künstliche Gewebe ein Hautmodell, insbesondere umfassend eine Dermis, umfassend mindestens eine erfindungsgemäße Basalmembran und eine Subcutis umfassend mindestens eine erfindungsgemäße Basalmembran, vorzugsweise ein unterschiedliche Basalmembran.

Bevorzugt sind die in dem erfindungsgemäßen Hautmodell eingesetzten mindestens zwei Basalmembranen spezifisch ausgebildet, insbesondere weisen sie jeweils mindestens eine Zelle eines spezifischen Zelltyps auf, insbesondere unterschiedlichen Zelltyps.

In bevorzugter Ausführungsform liegen die Zellen homogen verteilt innerhalb der erfindungsgemäßen Basalmembran vor.

Die vorliegende Erfindung betrifft auch therapeutische Anwendungen der erfindungsgemäßen Basalmembranen und diese aufweisenden künstlichen Gewebe.

Die vorliegende Erfindung betrifft auch Verfahren zur Therapie von, insbesondere erkrankten, Menschen oder Tieren, und Erzeugnisse zur Verwendung in solchen Verfahren.

Insbesondere kann die erfindungsgemäße Basalmembran als Wundeinlage, insbesondere zur Therapie von bevorzugt chronischen Wunden oder Brandwunden, insbesondere unter Einbringen, insbesondere Einlegen, in Wunden, Besiedelung mit Gewebezellen aus den Wundrändern, Verschließung der Wunde über dem erfindungsgemäßen Basalmembran durch Zellen, insbesondere Keratinozyten eingesetzt werden.

Die erfindungsgemäße Basalmembran kann auch als poröses Gewebe, welches in-vivo das Einwachsen von Zellen während der Regeneration nach zum Beispiel chirurgischen Eingriffen oder Resektionen in der (Krebs-)Therapie ermöglicht, verwendet werden.

Insbesondere betrifft die vorliegende Erfindung auch Verfahren zur Therapie von, insbesondere erkrankten, Menschen oder Tieren unter Verwendung des erfindungsgemäßen künstlichen Gewebes oder der Basalmembran und die Basalmembran oder das erfindungsgemäße künstliche Gewebe zur Verwendung in solchen Verfahren. Insbesondere kann das erfindungsgemäße künstliche Gewebe als ATMP (Advanced Therapy Medicinal Product) eingesetzt werden, in bevorzugter Ausführungsform besiedelt mit gewebespezifischen Zellen eines Ziel-Zelltyps, wobei der Einsatz in oder als stromalen bzw stromales Gewebe/Organ(en) bevorzugt ist, insbesondere in oder als Haut, Knorpel, Arterien und/oder Venen, Herz, Niere, Leber, Urogenitaltrakt, Atemwege, Knochen, Darm oder Cornea.

Die vorliegende Erfindung betrifft auch nicht-therapeutische Anwendungen der erfindungsgemäßen Basalmembran und diese aufweisenden künstlichen Gewebe, zum Beispiel kosmetische Anwendungen.

Im Zusammenhang mit der vorliegenden Erfindung wird unter dem Begriff "Faserzahl/mm²" die Anzahl der Fasern pro mm² Fasergelege verstanden, hier auch als Faserdichte bezeichnet, wobei sich die Fläche auf die gesamte Fläche der Ober- oder Unterseite des Fasergeleges in ihren flächigen Ausdehnungsrichtungen, also Länge und Breite, bezieht. Für die Flächenberechnung wird allein die Oberseite oder die Unterseite herangezogen, also nicht beide und auch nicht die Kantenflächen. Die Faserdichte wird bevorzugt Licht- oder Rasterelektronenmikroskopisch bestimmt.

Ausdehnungsrichtungen räumlicher Strukturen, wie Fasergelege, Trägerelement oder Basalmembran der vorliegenden Erfindung werden unter Bezugnahme auf ein kartesisches dreidimensionales Koordinatensystem als Länge, Breite und Dicke bezeichnet, wobei die Länge der x-Achse, die Breite der in der gleichen Ebene liegenden y-Achse und die Dicke der senkrecht, insbesondere vertikal, auf dieser aus x- und y-Achse aufgespannten Ebene stehenden z-Achse entspricht. Flächige Ausdehnungsrichtungen der genannten räumlichen Strukturen, insbesondere Länge und Breite, sind Richtungen, in denen die Ausdehnung deutlich größer, insbesondere vielfach größer, insbesondere mindestens 5-mal größer als in nicht-flächigen Ausdehnungsrichtungen, insbesondere Dicke, ist.

Im Zusammenhang mit der vorliegenden Erfindung wird die "pro Fläche des Trägerelements aufgebrachte Materialmenge" gemäß Beispiel 1 berechnet.

Im Zusammenhang mit der vorliegenden Erfindung wird die "Maschenfläche" des Fasergeleges bestimmt mittels Mikroskopieverfahren, insbesondere ohne Medium oder in Wasser, insbesondere ohne mechanische Belastung. Am geeignetsten ist dabei die Anwendung von Durchlichtmikroskopie oder von 3D-Methoden wie Laser-Scanning-Mikroskopie (LSM) oder der ähnlichen Methode konvokale Reflexionsmikroskopie. Aus dem entstandenen 3D-Bild wurden anschließend die Einzelbilder einer Nanofaserlage zu einem 2D-Bild übereinandergelegt. Anschließend wird die Fläche der Zwischenräume zwischen den Nanofasern bestimmt. Insbesondere wird die Maschenweite bestimmt gemäß der Verfahrensanweisung aus Beispiel 4.

Im Zusammenhang mit der vorliegenden Erfindung wird unter dem Begriff "Fasergelege", hier auch als Faservlies bezeichnet, ein Flächengebilde verstanden, das aus ungeordneten oder gezielt ausgerichteten Fasern besteht, wobei in bevorzugter Ausführungsform die Fasern nicht miteinander verbunden, verwoben oder verschlungen sind.

Im Zusammenhang mit der vorliegenden Erfindung wird unter dem Begriff "Seite" die flächig ausgebildete Seite eines Fasergeleges oder einer Basalmembran verstanden, insbesondere die Ober- oder Unterseite, wobei die Kantenflächen oder Stirnflächen der Membran oder des Fasergeleges aufgrund ihrer geringen Dickenausdehnung nicht mit dem Begriff "Seite" erfasst werden.

Im Zusammenhang mit der vorliegenden Erfindung wird unter dem Begriff "Elektrospinner" ist ein Gerät verstanden, das zur Elektrospinning-Technik verwendet wird, um Fasern sehr geringen Durchmessers aus einer Polymerlösung oder -schmelze herzustellen, insbesondere indem eine Hochspannung angelegt wird, um einen Polymer-Flüssigkeitstropfen in einen dünnen Strahl zu ziehen, der dann durch Verdunstung oder Erstarrung zu einer nanofaserigen Struktur umgewandelt wird. Eine Elektrospinner umfasst insbesondere eine Hochspannungsquelle, eine Spritzenpumpe zur Förderung der Polymerlösung mit kontrollierter Geschwindigkeit, eine Düse, zum Beispiel Nadel oder Kapillare, für den Austritt der Polymerlösung und einen Kollektor, zum Beispiel eine Metallplatte oder -trommel zum Auffangen und Ausformen der Fasern.

Im Zusammenhang mit der vorliegenden Erfindung wird unter dem Begriff "biokompatibel" verstanden, dass ein Material oder eine Substanz das Wachstum und die Funktionsfähigkeit von Zellen, insbesondere deren Fähigkeit, Proteine zu exprimieren und sie zu sezernieren, nicht beeinträchtigt, insbesondere dass das Material oder die Substanz vom menschlichen oder tierischen Körper akzeptiert wird, insbesondere ohne, dass bei Kontakt eine schädliche Immunreaktion oder toxische Wirkung ausgelöst wird. Biokompatible Materialen sind vorzugsweise nicht toxisch, nicht immunogen, nicht krebserregend und/oder je nach Anwendung stabil oder abbaubar.

Im Zusammenhang mit der vorliegenden Erfindung wird unter dem Begriff "biodegradierbar" verstanden, dass das betreffende Element, insbesondere Substanz, insbesondere Polymer, durch biologische Prozesse, insbesondere natürlicherweise vorkommende biologische Prozesse, insbesondere natürlicherweise in einem menschlichen oder tierischen Körper vorkommende biologische Prozesse, abgebaut werden kann und so seine strukturelle und/oder stoffliche Integrität verliert.

Im Zusammenhang mit der vorliegenden Erfindung wird unter dem Begriff "Zellkulturbehälter" bevorzugt eine Zellkulturplatte, Mikroplatte, Petrischale, Bioreaktor, Mikrofluidik-Chip, Multi-Chamber-System, Rollerflasche oder Zellkulturflasche verstanden.

Die erfindungsgemäßen Verfahren zeichnen sich durch eine bevorzugte Abfolge von Verfahrensschritten x) (optionales Beschichten mit Modifikationsmaterial), a), y1) (optionales Aufbringen von Stabilisierungsfasern auf Oberseite des Fasergeleges), z) (optionales Entnehmen aus Elektrospinner), b), v) (optionales Ablösen des Trägerelementes), y2) (optionales Aufbringen von Stabilisierungsfasern auf Unterseite des Fasergeleges nach Durchführung von Verfahrensschritt v)), c) und d) aus.

Die erfindungsgemäßen Verfahren zeichnen sich durch eine bevorzugte Abfolge von Verfahrensschritten x) (optionales Beschichten mit Modifikationsmaterial), a), y1) (optionales Aufbringen von Stabilisierungsfasern auf Oberseite des Fasergeleges), z) (optionales Entnehmen aus Elektrospinner), b), v) (optionales Ablösen des Trägerelementes), y2) (optionales Aufbringen von Stabilisierungsfasern auf Unterseite des Fasergeleges nach Durchführung von Verfahrensschritt v)), w) (Überführen in Zellkulturbehälter), c) und d) aus.

In besonders bevorzugter Ausführungsform finden die Verfahrensschritte, sofern nicht anders angegeben oder fachmännisch ersichtlich, in der in der vorliegenden Offenbarung angegebenen Reihenfolge statt.

In einer besonders bevorzugten Ausführungsform der vorliegenden Erfindung ist vorgesehen, dass zwischen den einzelnen explizit angegebenen Verfahrensschritten keine weiteren Verfahrensschritte durchgeführt werden.

Im Zusammenhang mit der vorliegenden Erfindung wird unter dem Begriff "und/oder" verstanden, dass alle Mitglieder einer Gruppe, welche durch den Begriff "und/oder" verbunden sind, sowohl untereinander kumulativ in einer beliebigen Kombination, als auch alternativ zueinander dargestellt sind. Exemplarisch ist für den Ausdruck "A, B und/oder C", folgender Offenbarungsgehalt darunter zu verstehen: i) (A oder B oder C), oder ii) (A und B), oder iii) (A und C), oder iv) (B und C), oder v) (A und B und C), oder vi) (A und B oder C), oder vii) (A oder B und C), oder viii) (A und C oder B).

Im Zusammenhang mit der vorliegenden Erfindung addieren Einzelkomponenten oder Bestandteile einer Gesamtheit, insbesondere der erfindungsgemäßen Basalmembran oder einer seiner Komponenten, die quantitativ in relativer Form, insbesondere in Prozentangaben bestimmt sind, sofern nicht anders angegeben, vorzugsweise auf 100 Gew.-% der jeweils in Bezug genommenen Gesamtheit beziehungsweise der Basalmembran oder, sofern in Bezug genommen, einer Komponente davon, auf.

Im Zusammenhang mit der vorliegenden Erfindung werden, sofern nicht anders angegeben, nicht angegebene Nachkommastellen einer Dezimalzahl als 0 verstanden.

Weitere vorteilhafte Ausgestaltungen der Erfindung ergeben sich aus den Unteransprüchen.

Die nachfolgenden Beispiele und die dazugehörigen Figuren erläutern die vorliegende Erfindung, ohne sie jedoch einzuschränken.

Die Figuren zeigen:

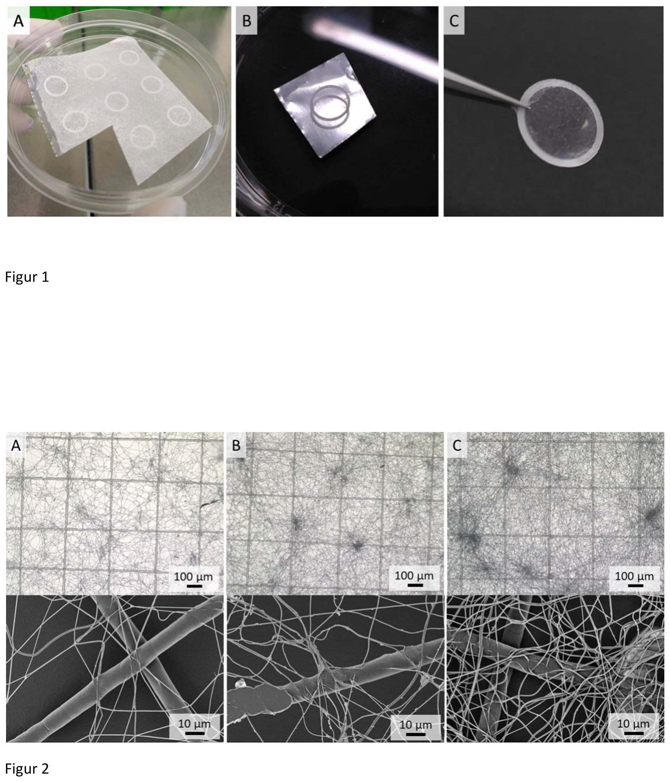

Figur 1 Handhabung des dünnen Fasergeleges. (A) Nach Herstellung des dünnen Fasergeleges erfolgte der Druck von PCL-Ringen direkt auf die Fasern. (B) Durch die wasserlösliche Zwischenschicht aus Modifikationsmaterial kann das Fasergelege mit dem Stabilisierungsrahmen nach kurzer Inkubation in Wasser abgelöst und (C) entnommen werden.
Figur 2 Erweiterung des dünnen Fasergeleges (Polyamid 6) mit MEW-Verstärkungsfasern (PCL, Polycaprolacton).

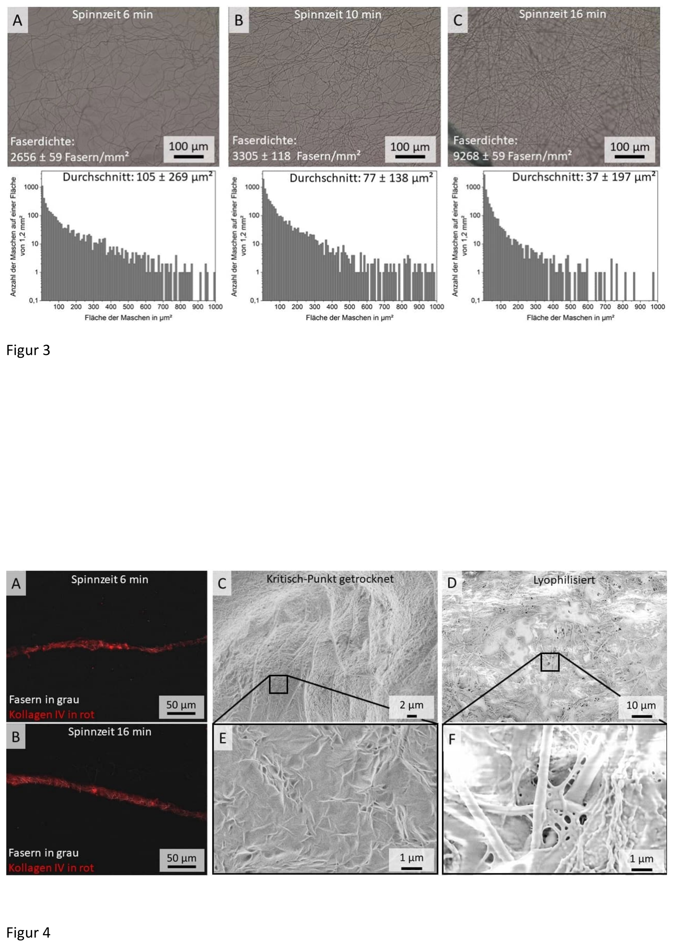

Figur 3 Charakterisierung der dünnen Fasergelege durch Messung der Maschenflächen.
Figur 4 Charakterisierung des zellulär modifizierten und dezellularisierten Fasergeleges. Im Querschnitt lassen sich die Fasern durch ihre charakteristische Form erkennen. Das immunhistologisch gefärbte Kollagen IV ist um und zwischen den Fasern deutlich erkennbar.

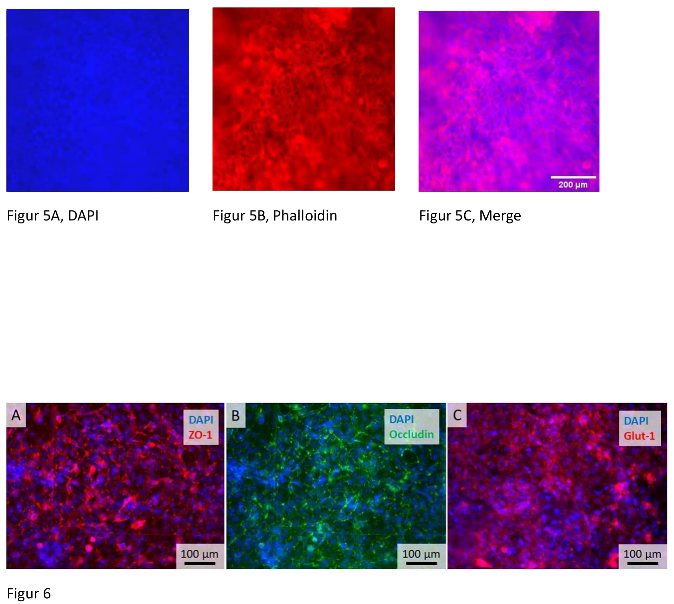

Figur 5 Dünndarmepithelzellen bilden Monolayer auf synthetischer erfindungsgemäßer Basalmembran. Die DAPI-Färbung (A) zeigt die charakteristischen runden Zellkerne mit einem Durchmesser von etwa 10 µm. Die Phalloidin-Färbung (B) beschreibt den Zellkörper über das Zytoskelett und färbt außer dem Zellkern den Rest der Zelle an. (C) beschreibt eine Überlagerung beider Färbungen.
Figur 6 HiPS-Zell-abgeleitete Endothelzellen der Blut-Hirn-Schranke bilden Monolayer auf der biologisierten erfindungsgemäßen synthetischen Basalmembran. Die DAPI-Färbung zeigt in allen Aufnahmen die charakteristischen runden Zellkerne mit einem Durchmesser von etwa 10 µm. Das cytoplasmatische Tight-Jungtion-Protein ZO-1 (A) ist um jeden Zellkörper in variierender Präsenz vorhanden. Ein weiteres Tight-Jungtion-Protein Occludin (B) beschreibt die Verbindung der Zellen und ist daher linienförmig um die einzelnen Zellen zu erkennen. Das Membran-Transporter-Protein Glut-1 (C) befindet sich auf der Oberfläche der Zellen und ist daher auf allen Zellen in variierender Präsenz erkennbar.

### Beispiele:

### Beispiel 1 - Grundgerüst aus elektrogesponnenen Fasern und Stabilisierungsrahmen

Die Herstellung der Fasergelege erfolgte in einem Elektrospinner mit einer rotierenden Walze des Durchmessers von 10,5 cm. Die Oberfläche wurde mit einer Aluminiumfolie als Trägerelement umwickelt und durch Klebeband eine definierte Oberfläche von 396 cm² eingestellt. Anschließend erfolgte die Beschichtung der Oberfläche mit dem Modifikationsmaterial NaCl-Pulver, um das Abnehmen der Fasern später zu vereinfachen (Verfahrensschritt x)). Für den Spinnprozess (Verfahrensschritt a)) wurde eine Polyamid 6-Lösung mit einer Konzentration von 12 % m/V in 1,1,1,3,3,3 Hexa-fluoro-2-propanol bereit gestellt und in einer Spritze mit dem Abstand Kanülenspitze - Oberfläche Walze von 15 cm platziert. Während des Spinnprozesses wurde an der Pumpe eine Fördergeschwindigkeit von 0,6 mL/h, eine Potentialdifferenz von 11 kV, eine Rotationsgeschwindigkeit von 100 U/min sowie eine parallele Bewegung der Kanüle zur Walze von 10 mm/s eingestellt. Die Spinndauer wurde in diesem Beispiel über 6 min, 10 min und 16 min variiert.

Aus diesen Parametern wird der theoretische Fasermaterialgehalt pro Fläche wie folgt bestimmt: Geförderte Masse / Oberfläche = (0,12 * 0,6 mL/h * 1,14 g/cm³ * Spinnzeit) / 396 cm².

Der Fasermaterialgehalt pro Fläche betrug: 20,7 µg/cm² (6 min), 34,5 µg/cm² (10 min), 55,3 µg/cm² (16 min).

Anschließend wurde das mit dem Stabilisierungsrahmen assoziierte Fasergelege auf dem Trägerelement in einem Verfahrensschritt z) aus dem Elektrospinner entnommen und in einem FDM-3D-Drucker (Fused Deposition Modeling (FDM)) platziert. Der Druck (Verfahrensschritt b)) der Stabilisierungsringe, also des Stabilisierungsrahmens, erfolgte mit Polycaprolacton mit einem Innendurchmesser von 9 mm und Außendurchmesser von 10 mm direkt auf die Fasern (Figur 1A). Abschließend kann das Konstrukt, also das mit dem Stabilisierungsrahmen assoziierte Fasergelege, von dem Trägerelement durch kurze Inkubation in Wasser abgelöst und entnommen werden (Verfahrensschritt v)) (Figuren. 1B, C).

### Beispiel 2 - Zusätzliche Stabilisierung des Fasergeleges über µm-Fasern

Für eine zusätzliche Stabilisierung können zusätzliche Fasern entweder direkt auf die elektrogesponnenen Fasern aufgebracht werden (Verfahrensschritt y1)) mit anschließender Bedruckung des Stabilisierungsrahmen oder, wie folgt beschrieben, nach der Bedruckung des Stabilisierungsrahmens auf die Unterseite des Konstruktes (Verfahrensschritt y2)). Dafür wurde das Konstrukt aus elektrogesponnenen Fasern und Rahmen mit Wasser von der Aluminium-Oberfläche gelöst (Verfahrensschritt v)), getrocknet und umgedreht in einem MEW-Drucker (Melt-Electro-Writing- (MEW)) platziert. Anschließend erfolgte die Bedruckung der elektrogesponnenen Fasern über MEW von Polycaprolacton in Form einer Schachbrettstruktur mit Faser-zu-Faser-Abständen von 300 µm und einer Faserdicke von ca. 10 µm (Verfahrensschritt y2)). Figur 2 zeigt die resultierende Struktur über verschiedene Mikroskopie-Methoden und Vergrößerungen. Figur 2 zeigt lichtmikroskopische Aufnahmen (oben) und REM (Rasterelektronenmikroskopie)-Aufnahmen (unten) mit verschiedenen elektrogesponnenen Faserdichten, hergestellt mit (A) 6 min, (B) 10 min und (C) 16 min Spinnzeit. Die im Vergleich zu den auch dargestellten Fasern des Fasergeleges deutlich durchmesserstärkeren Verstärkungsfasern wurden über Melt-Electro-Writing auf die Rückseite des Fasergeleges aufgebracht.

Diese Vorgehensweise erlaubt große Freiheiten in der Struktur und Anordnung dieser MEW-Fasern, insbesondere in Hinblick auf Faserdicken, Faserabstände, Winkelausrichtung der Fasern sowie nicht linear abgelegte Fasern.

### Beispiel 3 - Biologisierungsprozess des synthetischen Grundgerüst

Das synthetische Grundgerüst, also das Fasergelege, wurde zunächst in einer Transwell-Vorrichtung (eingespannt in eine Zellkrone, zwei Plastikzylinder mit unterschiedlichem Durchmesser, die ineinandergelegt werden) eingelegt, in einer 24-Well-Platte platziert (Verfahrensschritt w), Überführen in Zellkulturbehälter) und mit insgesamt 1,5 ml Kulturmedium außen und innen bedeckt. Anschließend erfolgte die Kultur (Verfahrensschritt c)) von 100 000 Zellen pro Probe für eine Dauer von 14 Tagen (Medienwechsel 3-mal pro Woche). Zum Entfernen der Zellen wurde zunächst das Medium abgenommen, die Probe mit einer Lösung aus Natriumdesoxycholat (0,33 g / 10 ml VE-Wasser; VE: vollentsalzt) bedeckt und für 30 min bei RT auf einem Schüttler inkubiert (Verfahrensschritt d)). Zum Entfernen der Zellreste erfolgten 4 Waschschritte mit PBS (Phosphatgepufferte Kochsalzlösung) für 20 min bei RT auf einem Schüttler und daraufhin die Inkubation über Nacht in frischem PBS- bei 4°C. Im letzten Schritt erfolgte die Entfernung der DNA durch Inkubation in einer DNAse-Lösung bei 37°C für 1 Stunde. Nach weiteren 3 Waschschritten in PBS wird das synthetische biologisierte Grundgerüst bei 4°C bis zur weiteren Anwendung gelagert. Alle Schritte wurden unter sterilen Bedingungen durchgeführt, um abschließende gewebeschädliche Sterilisationsmethoden zu vermeiden.

### Beispiel 4 - Maschen

Zur Charakterisierung der Faserdichten wurden an rasterelektronenmikroskopischen oder lichtmikroskopischen Aufnahmen der Fasergelege aus Beispiel 1 die Anzahl der Fasern/Fläche und die Maschenflächen bestimmt. Figuren 3 A-C (oben) zeigen beispielhaft die Faserdichten der drei verschiedenen Spinnzeiten. Die Darstellung der Maschenflächen beschreibt Figur 3A-C, unten. Figur 3 zeigt lichtmikroskopische Aufnahme der Fasergelege (oben) mit zugehörigem Histogramm der Maschenflächen und Anzahl (unten). Die Dichten der Fasergelege wurde eingestellt über die Spinnzeit von (A) 6 min, (B) 10 min und (C) 16 min. Die Bestimmung der Faserdichte erfolgte händisch beziehungsweise die Maschenfläche über ImageJ und ist dargestellt im Größenbereich zwischen 1 µm² und 1000 µm².

### Beispiel 5- biologisierte Basalmembran

Zur Untersuchung der Struktur und Zusammensetzung des fertigen Produktes der synthetisch basierten und biologisierten Basalmembran erfolgte zum einen eine Immunfluoreszenzfärbung gegen Kollagen Typ 4 von Paraffinschnitten. Figur 4A zeigt das Fasergelege mit 6 min Spinnzeit und Figur 4B mit 16 min Spinnzeit. Zum anderen wurden die Proben gemäß Figur 4B (16 min Spinnzeit) über Kritisch-Punkttrocknung (Figur 4C, E: für Kritisch-Punkttrocknung mit 2 und 1 µm-Maßstab) oder Lyophilisation (Figur 4D, F: für Lyophilisation mit 10 und 1 µm-Maßstab) getrocknet und dessen dichte Oberfläche über Rasterelektronenmikroskopie überprüft. Die Präsenz von Kollagen IV (rote Färbung) ist über der kompletten Fläche und Stärke des Fasergeleges erkennbar, durch Immunfluoreszenz von Paraffinschnitten; beispielshaft in der Probe mit (A) 6 min Spinnzeit und (B) 16 min Spinnzeit. Sowohl nach (C) Kritisch-Punkttrocknung als auch (D) Lyophilisation kann eine komplette Ausfüllung der Faserzwischenräume mit ECM-Matrix bestätigt werden (für 16 min Spinnzeit: Figuren 4C, D, E und F).

### Beispiel 6 - Dünndarm

Um die Anwendbarkeit der erfindungsgemäßen biologisierten Basalmembran bestätigen zu können, wurden humane primäre Dünndarmorganoide, kultiviert in Matrigel^{®} (3D Umgebung), zu Einzelzellen mit TrypLE Express dissoziiert und 400.000 Einzelzellen apikal auf die biologisierte Basalmembran appliziert. Zur Kultur der primären Organoide wird ein Mediumscocktail bestehend aus einer Vielzahl an Wachstumsfaktoren, die die Proliferation unterstützen, die Apoptose unterdrücken und die Differenzierung verhindern für einen Zeitraum von 5 Tagen verwendet, bis eine geschlossene Epithelzellschicht entsteht. Für weitere 5 Tage erfolgt die Applikation eines weiteren Mediumscocktails (tägliche Medienwechsel), der gezielt eine Differenzierung des Darmepithels induziert (Schweinlin et al., 2016, DOI: 10.1089/ten.TEC.2016.0101, Däullary et al., 2023, https://doi.org/10.1080/19490976.2023.2186109). Die Bestimmung der Bildung eines geschlossenen und einschichtigen Zelllayers wurde mittels Immunfluoreszenzfärbung nachgewiesen. Die Zellkerne wurden mittels DAPI (blau) und die Zellmembran mittels Phalloidin (rot) gefärbt (siehe Figur 5). Figur 5 zeigt repräsentative Bilder einer Immunfluoreszenzfärbung. Die Marker (A) DAPI (blau, Zellkerne) und (B) Phalloidin (rot, Zellmembran) zeigen Bildung eines einschichtigen, geschlossenen Epithelzelllayers auf der dezellularisierten Basalmembran. (C) zeigt einen Merge der beiden gefärbten Zellstrukturen.

### Beispiel 7 - Blut-Hirn-Schranke

Ein weiteres Anwendungsbeispiel der erfindungsgemäßen biologisierten Basalmembran stellt der Aufbau von in vitro Modellen/Testsystemen der humanen Blut-Hirn-Schranke/Neurovaskulären Einheit dar. Hierfür wurden aus hiPS-Zellen (humane iPS) gewonnene und differenzierte Endothelzellen der Blut-Hirn-Schranke an Tag 8 der Differenzierung A DAPI B Phalloidin C Merge (Appelt-Menzel et al., 2017, DOI: 10.1016/j.stemcr.2017.02.021) als Einzelzellen in einer definierten Aussaatdichte von 1x10⁶ Zellen/cm² Kulturfläche auf die biologisierte Basalmembran aufgebacht. Die Zellen wurden für 2 Tage mit zellspezifischem Medium behandelt, um die Ausbildung eines dichten, Barriere-bildenden Monolayers zu gewährleisten. Zur Charakterisierung des Differenzierungserfolgs der hiPS-Zell-abgeleiteten Endothelzellen der Blut-Hirn-Schranke erfolgten Immunfluoreszenzfärbungen auf gewebespezifische Tight Junction Proteine und Transporter (Figuren 6A-C). Figur 6 zeigt repräsentative Bilder einer Immunfluoreszenzfärbung für die Marker (A) ZO-1 (Zona Occludens), (B) Occludin und (C) Glut-1 (Glucosetransporter), welche die Bildung eines Monolayers auf der biologisierten Basalmembran und die Expression charakteristischer Blut-Hirn-Schranken-Marker zeigen.

## Patentansprüche

1. Verfahren zur Herstellung einer künstlichen Basalmembran umfassend ein Fasergelege, das in einer aus mindestens einem Basalmembranprotein aufgebauten Matrix eingebettet ist, umfassend die folgenden Verfahrensschritte:
a) Herstellen und Aufbringen eines Fasergeleges, umfassend Fasern mit einem Faserdurchmesser von 100 bis 2000 nm, mittels Elektrospinnen von einem Fasermaterial auf die Oberfläche eines in einem Elektrospinner angeordneten Trägerelements, wobei das Fasergelege eine Faserzahl von 1000 bis 30000 Fasern/mm² aufweist,
b) Aufbringen eines Stabilisierungsrahmens aus mindestens einem Polymer auf das auf dem Trägerelement angeordnete Fasergelege unter Bedingungen, die ein Assoziieren des Stabilisierungsrahmens mit dem Fasergelege erlauben,
c) Kultivieren des am Stabilisierungsrahmen assoziierten Fasergeleges mit in das Fasergelege eingebrachten Zellen mindestens eines Zelltyps, die zur Expression von mindestens einem Basalmembranprotein befähigt sind, unter Bedingungen, die die Expression des mindestens einen Basalmembranproteins erlauben und unter Erhalt einer aus dem mindestens einen Basalmembranprotein aufgebauten Matrix, in welcher das Fasergelege eingebettet ist und
d) Entfernen der Zellen des mindestens einen Zelltyps unter Erhalt der künstlichen Basalmembran.

2. Verfahren nach Anspruch 1, wobei in Verfahrensschritt b) der Stabilisierungsrahmen als Zellkulturbehälter ausgebildet wird und das in Verfahrensschritt c) vorgesehene Kultivieren der Zellen in dem als Zellkulturbehälter ausgebildeten Stabilisierungsrahmen durchgeführt wird.

3. Verfahren nach Anspruch 1, wobei im Anschluss an Verfahrensschritt b) das mit dem Stabilisierungsrahmen assoziierte Fasergelege in einem Verfahrensschritt w) in einen separaten Zellkulturbehälter überführt und in dem separaten Zellkulturbehälter Verfahrensschritt c) durchgeführt wird.

4. Verfahren nach einem der vorhergehenden Ansprüche, wobei zeitlich nach Verfahrensschritt b) in einem Verfahrensschritt v) das mit dem Stabilisierungsrahmen assoziierte Fasergelege von dem Trägerelement abgelöst wird.

5. Verfahren nach einem der vorhergehenden Ansprüche, wobei das Fasergelege eine durchschnittliche Maschenfläche von 10 bis 200 µm² aufweist oder die auf die Oberfläche des Trägerelements aufgebrachte Materialmenge der Fasern 10 bis 150 µg/cm² Oberfläche des Trägerelementes beträgt, oder beides.

6. Verfahren nach einem der vorhergehenden Ansprüche, wobei die Oberfläche des Trägerelementes vor Verfahrensschritt a) in einem Verfahrensschritt x) mit einem wasserlöslichen Modifikationsmaterial beschichtet wird.

7. Verfahren nach einem der vorhergehenden Ansprüche, wobei der Stabilisierungsrahmen in Verfahrensschritt b) mittels eines Druckverfahrens auf das Fasergelege aufgebracht wird.

8. Verfahren nach einem der vorhergehenden Ansprüche, wobei im Anschluss an Verfahrensschritt a) das das Fasergelege aufweisende Trägerelement in einem Verfahrensschritt z) aus dem Elektrospinner entnommen und in eine Vorrichtung zum Durchführen von Verfahrensschritt b) überführt wird.

9. Verfahren nach einem der vorhergehenden Ansprüche, wobei in einem Verfahrensschritt y) vor Verfahrensschritt c) Stabilisierungsfasern mit einem Faserdurchmesser von 1 bis 500 µm aus einem Stabilisierungsmaterial auf mindestens eine Seite des Fasergeleges aufgebracht werden.

10. Verfahren nach Anspruch 9, wobei die Stabilisierungsfasern in einem Verfahrensschritt y1) im Anschluss an Verfahrensschritt a) und vor Verfahrensschritt b) auf die Oberseite des auf dem Trägerelements befindlichen Fasergeleges aufgebracht werden.

11. Verfahren nach Anspruch 9, wobei die Stabilisierungsfasern in einem Verfahrensschritt y2) im Anschluss an das in Verfahrensschritt v) vorgesehene Ablösen des am Stabilisierungsrahmen assoziierten Fasergeleges von dem Trägerelement und vor dem in Verfahrensschritt c) vorgesehenen Kultivieren auf die Unterseite des Fasergeleges aufgebracht werden.

12. Verfahren nach einem der vorhergehenden Ansprüche, wobei Verfahrensschritt c) mindestens zweimal sequentiell mit Zellen unterschiedlicher Zelltypen durchgeführt wird.

13. Verfahren nach einem der vorhergehenden Ansprüche, wobei das Fasermaterial ausgewählt ist aus der Gruppe bestehend aus nicht degradierbaren Polymeren, insbesondere Polyether, Polyamide, Polyacrylnitril, Polystyrol, Polyethylenterephthalat, Polyoxazoline, chemisch funktionalisierte Polysaccharide und (Block-) Copolymere daraus, degradierbaren Polymeren, insbesondere Polyester, Poly-α-Hydroxysäuren, Polyglycolsäure, Polylactide, Polybutylsäuren, Polyurethane, Polypeptide und (Block-) Copolymere daraus, natürlichen Polymeren, insbesondere Polysacchariden, pflanzliche, tierische und menschliche Proteine, Lecithine, und anorganischen Materialien, insbesondere kondensiertes Tetraethylorthosilicat oder kondensierte Metallalkoholate, Silica-Gel, SiO₂, TiO₂, Bioglas und Kohlenstoff.

14. Verfahren nach einem der vorhergehenden Ansprüche, wobei der Zelltyp ausgewählt ist aus der Gruppe bestehend aus humanen Zelllinien, tierischen Zelllinien, primären humanen Zellen, primären tierischen Zellen, induzierten pluripotenten Zelllinien (IPS), IPS-abgeleiteten Zellen und stromalen Zellen.

15. Verfahren nach einem der vorhergehenden Ansprüche, wobei die künstliche Basalmembran 1 bis 20 µm dick ist.

16. Künstliche Basalmembran, umfassend ein Fasergelege aus elektroversponnenen Fasern mit einem Faserdurchmesser von 100 bis 2000 nm, wobei das Fasergelege in einer von Zellen mindestens eines Zelltyps, die zur Expression von mindestens einem Basalmembranprotein befähigt sind, erzeugten Matrix eingebettet ist, insbesondere hergestellt mittels eines Verfahrens nach einem der Ansprüche 1 bis 15.

17. Künstliche Basalmembran nach Anspruch 16, umfassend in oder auf die künstliche Basalmembran nach Anspruch 16 aufgebrachte Zellen mindestens eines Ziel-Zelltyps.

18. In vitro-Testsystem, Zellkultursystem, künstliches Gewebe, Implantat, Insert-System, Filtrationssystem oder Human-on-a-chip-/Organsystem, umfassend mindestens eine Basalmembran nach einem der Ansprüche 16 oder 17.
